# EUROPEAN PATENT APPLICATION

(11) **EP 1 946 723 A1**
(43) Date of publication of application: **23.07.2008**
(21) Application number: 05804074.2
(22) Date of filing: 31.10.2005
(51) Int. Cl.: A61F 2/14

(54) **SCLERAL BUCKLE BAND AND METHOD FOR MAKING IT**

(71) Applicant: Life Spring Biotech Co., Ltd., Taipei City (TW)
(72) Inventor: YEN, Hsiaocheng, Taiwan (CN); HSIAO, Joyi, Taiwan (CN); LEE, Wenhao, Taiwan (CN)
(74) Representative: Viering, Jentschura & Partner
(86) International application number: PCT/CN2005/001801
(87) International publication number: WO 2007/051345

(57) **Abstract**

A scleral buckling band and a method for making the same are described. The scleral buckling band is used for an ophthalmic operation, which is biocompatible and has a slender cylindrical structure formed by a decomposable and absorbable material. When being implanted into human body, the scleral buckling band is degraded and absorbed by the human body, without causing any immune response. After the patient is recovered, the scleral buckling band does not need to be taken out through another operation. Meanwhile, the decomposition rate of the scleral buckling band within the human body can be controlled through different preparation manners, so as to cater to different recovery speeds of different patients. Furthermore, the scleral buckling band contains different medicine, and after being implanted into human body and being decomposed, the scleral buckling band releases different specific medicine as time elapsed.

## Description

### BACKGROUND OF THE INVENTION

### Field of the Invention

The present invention relates to a scleral buckling band and a method for making the same, in particular, to a scleral buckling band used for an ophthalmic operation, which is biocompatible and capable of being decomposed and adsorbed by human body.

### Related Art

Retinal detachment is considered as the main reason for causing blindness. Due to retinal detachment, the retinal photoreceptor cells cannot obtain nutrition from the choroids. If the detachment lasts for a long time, the retinal atrophy may occur, which results in blindness. Currently, the methods used for clinically curing the retinal detachment mainly include operation, laser, freezing, or repair of pneumatic retinopexy, which aim at recovering the detached retina and curing the retinal tears, and meanwhile firmly joining the retina and choroids together.

In the above and relevant methods for curing retinal detachment, a scleral buckling operation is needed. Currently, most of the scleral buckling bands 1' used in the operation are made of silicone and hydrogel materials. However, such buckling materials cannot be decomposed and absorbed by human body, and even causes rejection response. Therefore, when the patient is recovered, the scleral buckling band has to be taken out through another operation, which causes many inconveniences and resource waste.

Accordingly, till now, it still needs a scleral buckling band that is decomposed and absorbed by human body naturally after being implanted into human body, without causing any immune response and without the problem of rejection response or requiring another operation for removing the implant.

### SUMMARY OF THE INVENTION

In order to solve the problems in the prior art, the present invention is directed to a scleral buckling band used for an ophthalmic operation. The scleral buckling band has a slender cylindrical structure formed by a biocompatible, decomposable, and absorbable material. After being implanted into human body, the scleral buckling band is degraded and absorbed by the human body, without causing any immune response. The present invention is further directed to a scleral buckling band containing medicine, which releases specific medicaments required by different periods of treatment as time elapsed.

The present invention provides a scleral buckling band containing medicine. The medicine includes an anti-microbial agent, an anti-inflammatory agent, a guided tissue growth factor, or another suitable medicine. The scleral buckling band carries specific medicaments according to the patients' different requirements, which enhances the medicament delivery efficiency.

The scleral buckling band of the present invention is made by combining collagens having different strengths with different medicaments to be contained in the scleral buckling band. Since different collagens with different strengths have different decomposition rates and different medicaments are contained, the scleral buckling band releases specific medicaments required in different periods of treatment as time elapsed, which thus enhances the barrier effect, and improves the effects of wound healing and tissue regeneration.

The present invention is further directed to a method for making a scleral buckling band.

In order to achieve the above objects, the present invention provides a scleral buckling band with different strengths, which is made of collagens having different strengths. The collagens having different strengths are prepared according to the content of collagens, type and cross-linking manner of the cross-linker.

The scleral buckling band of the present invention used for an ophthalmic operation is degraded and absorbed by human body after being implanted into the human body, without causing any immune response. In addition, the scleral buckling band does not need to be taken out through another operation after the patient is recovered, and it further carries specific medicaments according to different demands of the patients, which improves the delivery efficiency of the medicaments.

### BRIEF DESCRIPTION OF THE DRAWINGS

The present invention will become more fully understood from the detailed description given herein below for illustration only, which thus is not limitative of the present invention, and wherein:
FIG. 1 is a schematic structural view of a scleral buckling band according to the present invention;
FIG. 2 is a coordinate graph of tensile strength test results of the scleral buckling band according to the present invention;
FIG. 3 shows a picture of a microscopic structure of the scleral buckling band according to the present invention;
FIG. 4 shows a picture of animal experiment of the scleral buckling band according to the present invention;
FIG. 5 shows a picture of animal experiment of a scleral buckling band in the prior art; and
FIG. 6 is a bar graph about distortions of eyeballs through implanting the scleral buckling band of the present invention comprised with that in the prior art.

### DETAILED DESCRIPTION OF THE INVENTION

In order to make the main technical features and functions of the present invention more comprehensible, the present invention is further described below in detail with reference to the accompanying drawings.

### Preparation of Type I Collagen

1. A bovine skin and tendon enriched with collagens were cut into cubes with a size of about 0.5 cm³, placed into 10 L 95% alcohol, and stirred at 4°C for 24 h. The bovine tendon was taken out of the 95% alcohol, placed into 10 L 0.5 M acetic acid solution, and then stirred at 4°C for 72 h. Then, pepsin (SIGMA P7000, 4000 unit/ml) was added, and the solution was continuously stirred at 4°C for 24 h.
2. The mixture of Step 1 was filtered with a stainless steel mesh to remove the undecomposed residues. Sodium chloride was added continuously till the concentration thereof reached 1.0 M, and then the solution was stirred at 4°C for 30 min and centrifuged at a rotation speed of 10,000 g (Beckman Avanti J-20) for 30 min.
3. After removing the supernatant, 10 L 50 mM Tris-HCl buffer (pH 7.4) was added, and stirred at 4°C for 30 min. Then, sodium chloride was added again till the concentration thereof reached 4.0 M. The solution was stirred at 4°C for 30 min, and then centrifuged at a rotation speed of 10,000 g for 30 min.
4. After removing the supernatant, 10 L 50 mM Tris-HCl buffer (pH 7.4) was added, and stirred at 4°C for 30 min. Then, sodium chloride was added again till the concentration thereof reached 2.5M. The solution was stirred at 4°C for 30 min, and then centrifuged at a rotation speed of 10,000 g for 30 min.
5. After removing the supernatant, 5 L of a mixed solution of isopropanol and pure water (Isopropanol : H₂O = 1 : 4) was added, stirred at 4 °C for 30 min, and then centrifuged at a rotation speed of 10,000 g for 30 min. This step was repeated twice.
6. After removing the supernatant, 5 L 0.05M acetic acid solution was added, and stirred thoroughly and uniformly and then placed at a temperature of -90°C for being frozen. Then, the congelation was dried with a freeze dryer to a constant weight. The resulted dry product is type I collagen.

### Preparation of Scleral Buckling Band

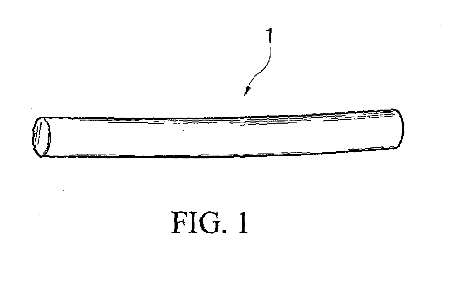

1. The type I collagen was added into a weak acidic aqueous solution (for example, 0.05 M aqueous acetic acid solution), and stirred at a high speed into a homogenous slurry, in which the slurry contains the type I collagen at a concentration of about 2 wt%. Then, glycosaminoglycans (GAGs) were dissolved into the weak acidic aqueous solution (for example, 0.05 M aqueous acetic acid solution) to get an aqueous solution at a weight concentration of about 0.4 wt%-1.0 wt%.
2. The aqueous GAGs solution was mixed with the type I collagen slurry, in which the ratio of the GAGs was about 2.0 wt%-5.0 wt% based on the weight of the type I collagen, and then placed at room temperature for 48 h to remove a part of the moisture, so as to get a thicker slurry mixture. Then, the resulted slurry mixture was made to pass through needle heads with a diameter of 0.9 mm and 0.5 mm to become homogenous. Then, the mixture was filled into a mold for making the scleral buckling band. Sequentially, a vacuum freeze-drying treatment was performed for about 36 h to remove the moisture in the solution. Then, the mixture was heated at 105°C in vacuum for 24 h, so as to be thermal dehydrated and cross-linked, and then, the 260 nm UV cross-link treatment was performed to get a predetermined scleral buckling band. Thereafter, a cross-link treatment was performed with a natural cross-linker (genipin) or glutaraldehyde, such that the collagen and the GAGs were cross-linked, so as to enhance the mechanical strength of the scleral buckling band, thereby the decomposition rate thereof was controlled. Finally, the scleral buckling band was washed with pure water and freeze dried to get a scleral buckling band with a slender cylindrical structure, as shown in FIG. 1.

### Mechanical Property Test of Scleral Buckling Band

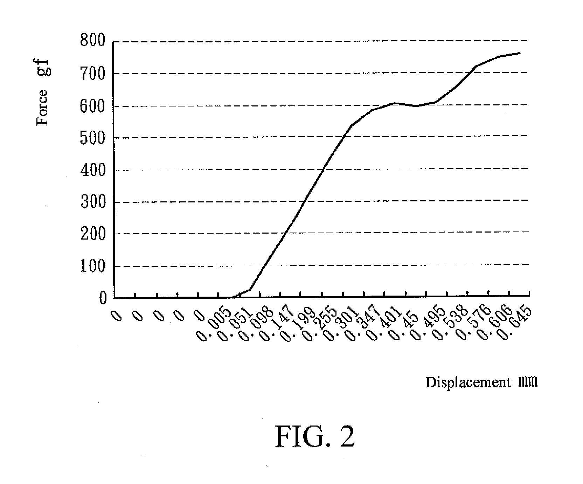

The resulted scleral buckling band (with a length of 5.0 cm, a diameter of 0.2 cm) is fixed on clamps of a material testing machine. The test length of the scleral buckling band confined between two clamps is 3.0 cm, and then a force is applied to stretch the scleral buckling band, till the scleral buckling band is broken. The force applied at the instance when the scleral buckling band is broken is recorded, and the tensile strength of the scleral buckling band is calculated as 2.37 MPa, as shown in FIG. 2.

### Observation of the Scleral Buckling Band with Microscope

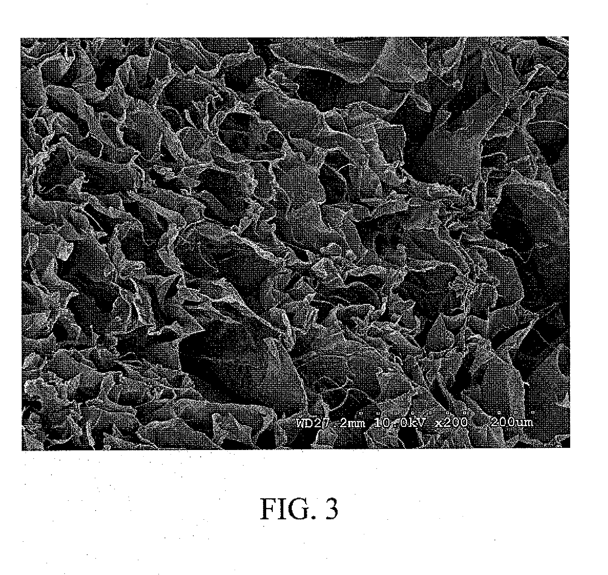

The resulted scleral buckling band is observed with a scanning electronic microscope (SEM), and the average porosity of the scleral buckling band is calculated to be about 120±40 µm, as shown in FIG. 3.

As polymer materials are easily processed and have high mechanical strength and desirable biocompatibility, they have been widely used to develop thousands of medical products, such as disposable medical equipments and implantable biomedical materials in the past decade.

Natural polymers include collagen and polysaccharide biopolymer, for example, hyaluronic acid (HA) and chitosan, and chemical synthetic polymers include polylactic acid (PLA), polyglycolic acid (PGA), and polylactic acid-glycolic acid (PLGA).

The PLA, PGA, and PLGA are all bio-absorbable polymer materials with desirable biocompatibility, and their co-polymers are decomposed into small molecular chain segments in a living body, and then discharged out of the living body as the metabolism process continues. Therefore, the PLA, PGA, and PLGA have desirable biocompatibility, bio-absorbability, and capable of being discharged out of the body as the metabolism process continues, which thus can be processed into different types of implantable biomedical materials with slow degradability and capable of releasing different coated medicaments.

HA is a polysaccharide biopolymer and widely exists in connective tissues, mucous tissues of vertebrates, crystalline lens of eyeballs, and capsules of some bacteria in the nature. Regardless of the source, the chemical composition and structure of HA are the same. Therefore, if HA is used as a medical polymer material, it can be decomposed and absorbed by the living body, without causing any immune response. Recently, HA has been gradually developed for the applications of post-operation tissue anti-adhesion and medicament release.

Chitosan is a biopolymer prepared by extracting from natural biological organism, and mostly exists in crustacean, which has desirable biocompatibility with cells of biological organisms, has no toxicity, and can be decomposed by biological organisms, and thus it can be developed as a carrier for drug release.

Collagen is a polymer for forming various extra-cellular matrixes and serves as combination tissues in animal cells. Collagen mainly exists in the form of an insoluble fibrin, and takes about 25%-30% of the proteins in human body. Therefore, the collagen has desirable biocompatibility, and can prevent human body from generating rejection response and can be absorbed by the tissues after being decomposed. The collagen can be extracted and purified from biological tissues. Then, the mechanical strength of the material can be enhanced by physical or chemical cross-linking treatment, and can also be made into a porous structure, which is suitable for being used as a temporary tissue filling material and can also be made into a base material for various artificial tissues.

According to the present invention, the material for making the scleral buckling band includes, but not limited to, collagen, PLA, PGA, PLGA, PCL, HA, and chitosan, and another polymer material. Preferably, the material is collagen.

According to the present invention, the scleral buckling band may have different strengths, and when an oculist performs an operation, the scleral buckling bands with different strengths may be selected to control the residence time of the scleral buckling band within the human body, so as to cater to different recovery speeds of different patients. It is known to those of ordinary skill in the art that, collagens with different strengths can be made according to the content of collagens, type and cross-linking manner of the cross-linker. However, the present invention is not intended to limit the content of collagens, type, and cross-lining manner of the cross-linker. The conventional methods for making collagens with different strengths all can be used in the present invention for making the scleral buckling band.

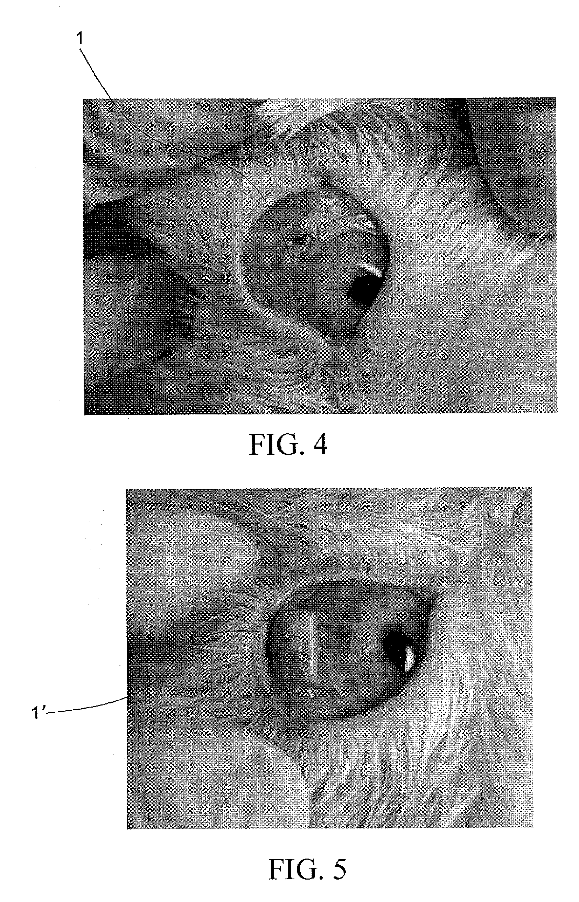

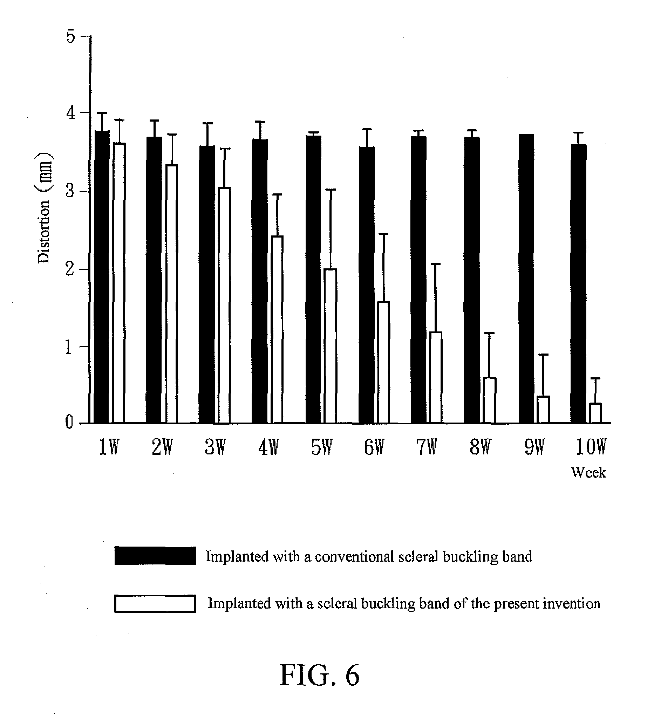

Referring to FIGs. 4 and 5, the distortion of eyeballs is measured by sonography as time elapsed. As the conventional scleral buckling band used in the operation is made of silicon, it cannot be metabolized and decomposed by enzymes and other substances within the human body, so that the buckling effect generated on the sclera after the scleral buckling band is implanted lasts for a long time, and the eyeball maintains a certain distortion, as a result, the vision is affected. The scleral buckling band of the present invention is mainly made of collagens and is metabolized and decomposed by the enzymes within the body, and accordingly, after the scleral buckling band is implanted, the buckling effect on the sclera is decreased as time elapsed. Besides achieving the functions of the conventional scleral buckling band, the scleral buckling band further makes the distortion of the eyeball be decreased as time elapsed, which means that the eyeballs return to the original shape, as shown in FIG. 6, so that the scleral buckling band of the present invention does not bring any influences on vision.

According to the present invention, the scleral buckling band optionally contains medicaments, such as an anti-microbial agent, an anti-inflammatory agent, a growth factor, or another suitable medicine. It is well known to those skilled in the art that, the polymer biomedical material is characterized in containing therapeutic or preventive medicaments therein when being prepared. The common biomedical materials are generally applied on parts under operation or wounds, and carry specific medicaments according to different requirements of different patients through the function of carrying drugs, so as to enhance the delivery efficiency of the medicaments.

According to the present invention, the scleral buckling band is made by combining collagens with different strengths and contains different medicaments, and since the collagens with different strengths have different decomposition rates and different medicaments are contained therein, the scleral buckling band releases specific medicaments required by different periods of treatment as time elapsed, which thus enhances the barrier effect, and improves the effects of wound healing and tissue regeneration.

In view of the above, the scleral buckling band of the present invention can be industrialized and has novelty and inventive step, and thus meets the patent requirements. The above description is merely a preferred embodiment of the present invention, but not intended to limit the scope of the present invention.

## Claims

1. A scleral buckling band, used for an ophthalmic operation, comprising a slender cylindrical structure formed by a biocompatible material.

2. The scleral buckling band according to claim 1, wherein the biocompatible material is selected from a group consisting of collagen, polylactic acid (PLA), polyglycolic acid (PGA), polylactic acid-glycolic acid (PLGA), polycaprolactone polyol (PCL), hyaluronic acid (HA), and chitosan.

3. The scleral buckling band according to claim 1, wherein the biocompatible material is collagen.

4. The scleral buckling band according to claim 3, wherein a strength of the scleral buckling band is adjusted according to a content of collagens, type and cross-linking manner of a cross-linker, so as to control a residence time of the scleral buckling band within the human body.

5. The scleral buckling band according to claim 1, further containing medicine.

6. The scleral buckling band according to claim 1, further containing specific medicaments released in different periods of treatment as time elapsed.

7. The scleral buckling band according to claim 6, wherein the specific medicament is an anti-microbial agent, an anti-inflammatory agent, or a guided tissue growth factor, or another suitable medicine.

8. A method for making a scleral buckling band, at least comprising:
preparing a biocompatible material;
filling the biocompatible material into a mold;
performing a vacuum freeze-drying treatment to remove moistures in the biocompatible material; and
performing a ultraviolet (UV) cross-linking treatment to obtain a predetermined slender cylindrical structure.

9. The method for making a scleral buckling band according to claim 8, wherein the biocompatible material is selected from a group consisting of collagen, PLA, PGA, PLGA, PCL, HA, and chitosan.

10. The method for making a scleral buckling band according to claim 8, wherein the biocompatible material is formed by mixing an aqueous solution of glycosaminoglycans (GAGs) with a collagen slurry, and then placing the mixture at room temperature to remove a part of the moisture in the mixture, so as to get a slurry mixture, wherein the ratio of the glycosaminoglycans is about 2 wt%-5 wt% based on a weight of the collagen.
